Europäisches Patentamt

European Patent Office

Office européen des brevets

(11) Veröffentlichungsnummer: **0 480 315 A2**

# EUROPÄISCHE PATENTANMELDUNG

(21) Anmeldenummer: **91116917.5**

(22) Anmeldetag: **04.10.91**

(51) Int. Cl.5: **A61K 37/02**, A61K 31/71,
A61K 31/675, A61K 31/535,
//(A61K37/02,31:535),
(A61K31/71,31:535),
(A61K31/675,31:535),
(A61K31/535,31:505)

(30) Priorität: **12.10.90 CH 3285/90**

(43) Veröffentlichungstag der Anmeldung:
**15.04.92 Patentblatt 92/16**

(84) Benannte Vertragsstaaten:
**AT BE CH DE DK FR GB IT LI LU NL SE**

(71) Anmelder: **F. HOFFMANN-LA ROCHE AG**
**Postfach 3255**
**CH-4002 Basel(CH)**

(72) Erfinder: **Teelmann, Kampe, Dr.**
**Marschalkenstrasse 72**
**CH-4054 Basel(CH)**

(74) Vertreter: **Grossner, Lutz, Dr. et al**
**Grenzacher Strasse 124 Postfach 3255**
**CH-4002 Basel(CH)**

(54) **Verwendung eines Retinoids.**

(57) Verwendung des 4-[2-[p-[(E)-2-(5,6,7,8-Tetrahydro-5,5,8,8-tetramethyl -2-naphthyl)propenyl]phenoxy]äthyl]-morpholins als Wirkstoff bei der Herstellung von pharmazeutischen Präparaten zur Unterstützung der Therapie mit einem Cytostatikum.

EP 0 480 315 A2

Eine Therapie mit Cytostatika ist häufig mit unerwünschten Wirkungen verbunden, die ihre Ursache in der Toxizität der Cytostatika haben. Beispiele solcher unerwünschter Wirkungen sind Haarausfall, Gewichtsverlust bzw. Appetitlosigkeit, Knochenmarksschädigungen, Magen-Darm-Störungen, Herzmuskelveränderungen, Hodenveränderungen, Leber- und Nierenschädigungen.

Es wurde nun gefunden, dass diese unerwünschten Wirkungen bei der Therapie mit Cytostatika vermindert oder vermieden werden können, wenn gleichzeitig oder zeitlich abgestuft die Verbindung 4-[2-[p-[(E)-2-(5,6,7,8-Tetrahydro-5,5,8,8-tetramethyl-2-naphthyl)propenyl]phenoxy]äthyl]morpholin (nachfolgend als "Verbindung Z" bezeichnet) verabreicht wird.

Die vorliegende Erfindung betrifft somit die Verwendung des 4-[2-[p-[(E)-2-(5,6,7,8-Tetrahydro-5,5,8,8-tetramethyl-2-naphthyl)propenyl]phenoxy]äthyl]morpholins als Wirkstoff bei der Herstellung von pharmazeutischen Präparaten zur Unterstützung der Therapie mit einem Cytostatikum. Die Erfindung betrifft weiterhin Erzeugnisse, enthaltend 4-[2-[p-[(E)-2-(5,6,7,8-Tetrahydro-5,5,8,8-tetramethyl-2-naphthyl)propenyl]phenoxy]äthyl]morpholin und ein Cytostatikum als Kombinationspräparat zur gleichzeitigen, getrennten oder zeitlich abgestuften Anwendung in der Krebsprophylaxe oder -Therapie sowie eine Handelspackung, enthaltend als pharmazeutischen Wirkstoff 4-[2-[p-[(E)-2-(5,6,7,8-Tetrahydro-5,5,8,8-tetramethyl-2-naphthyl)propenyl]phenoxy]äthyl]morpholin zusammen mit Instruktionen für dessen Verwendung in Kombination mit einem Cytostatikum zur gleichzeitigen, getrennten oder zeitlich abgestuften Anwendung in der Krebsprophylaxe oder -Therapie.

Verbindung Z, ihre Herstellung und Verwendung als Heilmittel, z.B. als Krebstherapeutikum, ist in der europäischen Patentpublikation A-2 0 331 983 beschrieben.

Cytostatika, deren unerwünschte Wirkungen durch eine gleichzeitige oder zeitlich abgestufte Therapie mit der Verbindung Z verringert oder vermieden werden können, sind solche vom Typ der alkylierenden Substanzen, wie z.B. Cyclophosphamid; Platinderivate; Antimetaboliten, z.B. Folsäure-Analoge wie Methotrexat, oder Pyrimidinderivate, wie 5-Fluoruracil; Anthracycline, wie Doxorubicin; Vinca-Alkaloide, wie Vinblastin oder Vincristin; sowie "Biological Response Modifiers" (BRM), wie Interleukine, z.B. IL-2, Interferone, z.B. IFN$\alpha$ und IFN-$\gamma$, Colony Stimulating Factors, z.B. G-CSF, GM-CSF und M-CSF, und Retinoide, z.B. Temaroten. Die Verminderung der unerwünschten Wirkungen wird erreicht, indem die oben beschriebenen Substanzen durch Kombination mit Verbindung Z in geringeren Dosen verabreicht werden können ohne die Antitumorwirkung zu vermindern.

Weiterhin wurde gefunden, dass die gleichzeitige Verabreichung der Verbindung Z und Interleukin oder Doxorubicin das Tumorwachstum in mehr als additivem Mass hemmt. In Kombination mit Cyclophosphamid wurde die Toxizität von CY weitgehend aufgehoben.

Diese unerwarteten Effekte bei der Tumorbehandlung mit der Verbindung Z und anderen Cytostatika wird durch die nachstehenden Versuchsresultate dokumentiert:

A. Behandlung von chemisch induzierten Mamma-Tumoren bei der Ratte mit Cyclophosphamid und Verbindung Z.

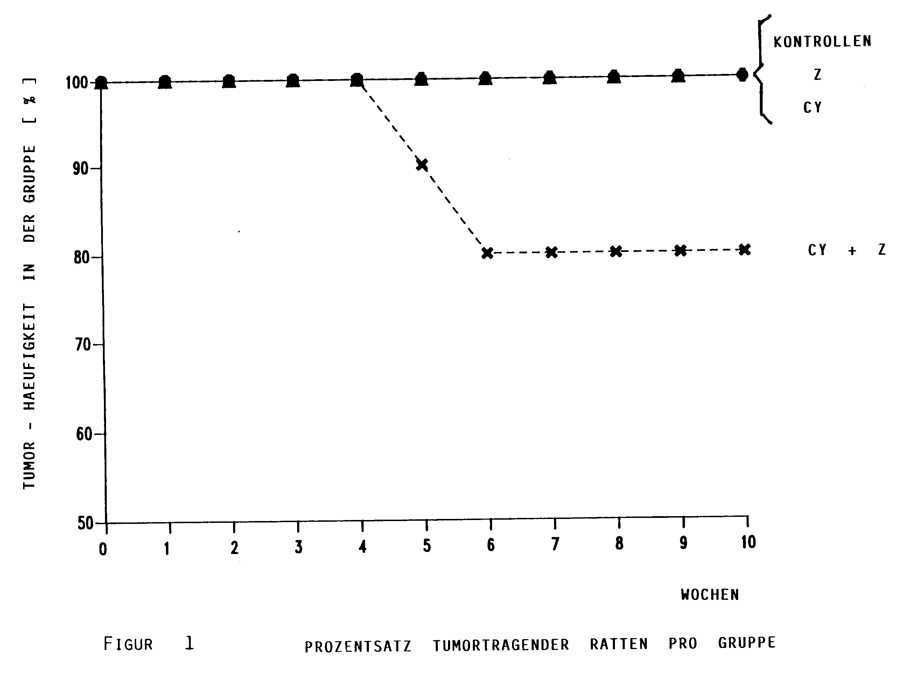

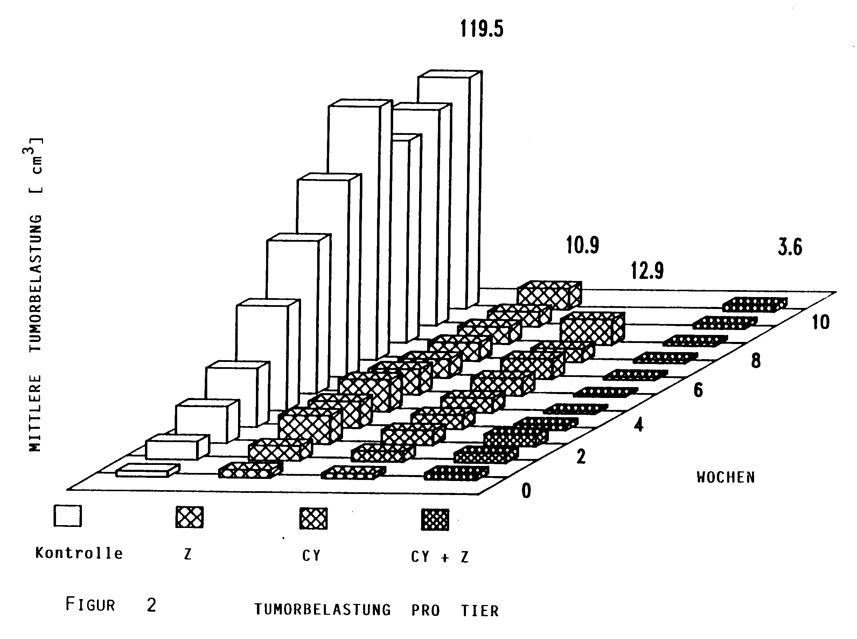

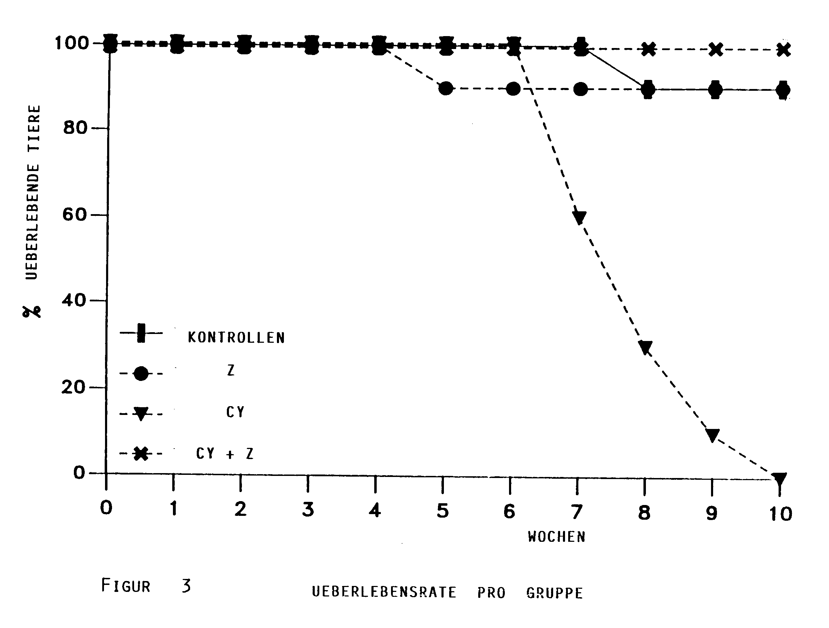

Weibliche Ratten erhielten im Alter von 50 Tagen oral eine Einzeldosis von 12 mg 7,12-Dimethylbenz-(a)anthracen in 0,5 ml Erdnussöl. Nach etwa 8 Wochen wurden die Ratten, die Mamma-Tumoren entwickelt hatten, in Gruppen zu 10 eingeteilt. Eine Gruppe erhielt nur Futter ohne Zusatz von Testsubstanz; eine Gruppe erhielt 75 mg/kg/Tag (7 mal wöchentlich) Verbindung Z als Futterzusatz; eine Gruppe erhielt 10 mg/kg/Tag Cyclophosphamid (5 mal wöchentlich) intraperitoneal; eine Gruppe erhielt Verbindung Z und Cyclophosphamid in den angegebenen Dosierungen. Die Behandlung erstreckte sich über 10 Wochen. Danach wurden Anzahl der Tumoren, Tumorvolumen und Mortalität bestimmt. Die Resultate sind aus Tabelle 1 und Figur 1-3 ersichtlich.

## Tabelle 1

| Gruppe | Verbindung(en) | Tumor-häufigkeit in der Gruppe [%] | Mittlere Anzahl Tumore/Ratte | Mittlere Tumor-belastung [cm³] | Anzahl der überlebenden Ratten | Körpergewicht [g] |
|---|---|---|---|---|---|---|
| **Versuchsbeginn** | | | | | | |
| 1 | Kontrolle | 100 | 2.8 ± 0.5 | 2.8 | 10 | 251 ± 5 |
| 2 | Z | 100 | 3.1 ± 0.5 | 4.0 | 10 | 256 ± 4 |
| 3 | Cy | 100 | 2.4 ± 0.4 | 2.6 | 10 | 266 ± 6 |
| 4 | Cy + Z | 100 | 2.7 ± 0.4 | 3.2 | 10 | 252 ± 5 |
| **nach 6 Wochen** | | | | | | |
| 1 | Kontrolle | 100 | 6.0 ± 0.8 | 100.2 | 10 | 267 ± 10 |
| 2 | Z | 100 | 4.0 ± 0.6 | 9.6 | 9 | 246 ± 6 |
| 3 | Cy | 100 | 5.7 ± 0.5 | 10.3 | 10 | 249 ± 5 |
| 4 | Cy + Z | 80 | 1.7 ± 0.4 | 1.5 | 10 | 243 ± 5 |
| **nach 10 Wochen** | | | | | | |
| 1 | Kontrolle | 100 | 6.6 ± 0.8 | 119.5 | 9 | 285 ± 8 |
| 2 | Z | 100 | 3.4 ± 0.6 | 10.9 | 9 | 255 ± 6 |
| 3 | Cy | NA | NA | NA | 0 | NA |
| 4 | Cy + Z | 80 | 2.8 ± 0.8 | 3.6 | 10 | 253 ± 5 |

NA = nicht anwendbar

Die Resultate zeigen den Tumor-hemmenden Effekt der beiden Testverbindungen, der bei kombinierter Verabreichung additiv erscheint. Ueberraschend zeigte sich, dass alle Versuchstiere, die Cyclophosphamid und die Verbindung Z erhalten hatten, die gesamte Versuchsdauer überlebten, während die alleinige Verabreichung von Cyclophosphamid zum vorzeitigen Tod aller Versuchstiere führte.

Fig. 1 zeigt die Tumorhäufigkeit ausgedrückt als Prozentsatz der Tumor-tragenden Tiere einer Gruppe.

Fig. 2 zeigt die mittlere Tumorbelastung pro Tier, die das Produkt aus der mittleren Anzahl der Tumore und dem mittleren Tumorvolumen pro Ratte darstellt.

Fig. 3 zeigt die Ueberlebensrate der Versuchstiere. Alle Tiere, die mit der Kombination von Z und Cyclophosphamid behandelt wurden, überlebten bis zum Versuchsende, wogegen in der Cyclophosphamid-behandelten Gruppe alle Tiere unter Symptomen von Anämie, Schwäche und Gewichtsverlust vorzeitig starben. Diese Daten zeigen, dass Z die Toxizität von Cyclophosphamid deutlich verringerte.

B. Behandlung von chemisch induzierten Mamma-Tumoren bei der Ratte mit Interleukin-2 und Verbindung Z.

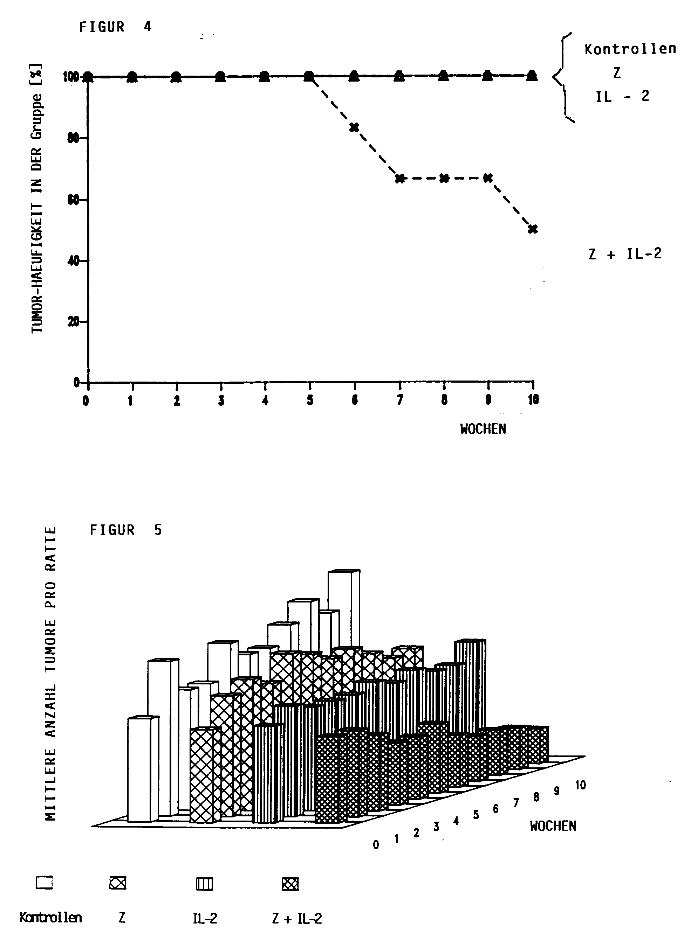

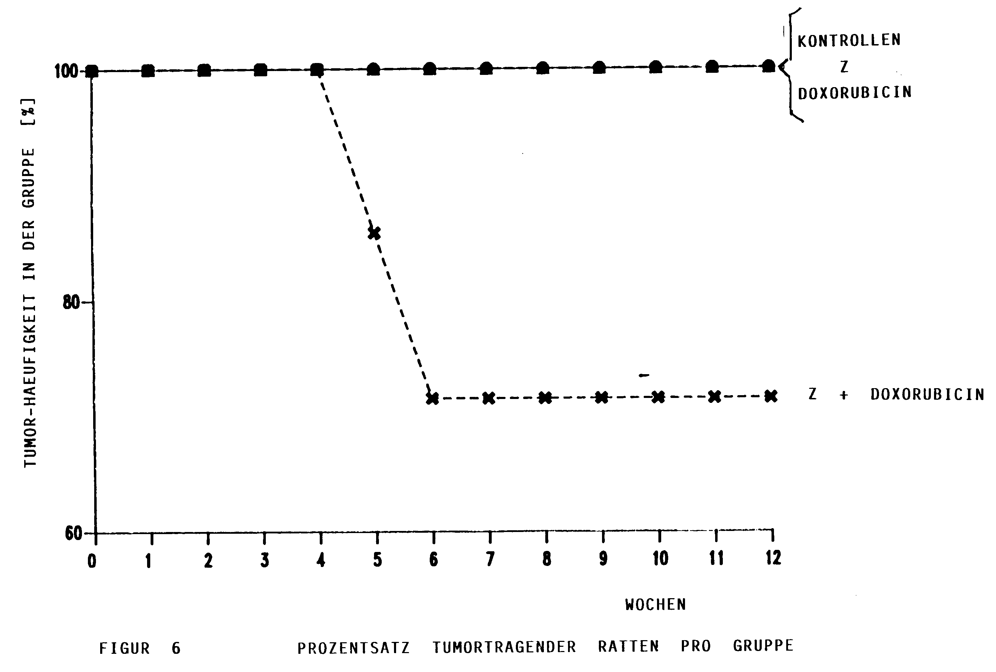

Weibliche Ratten mit wie unter A. beschrieben induzierten Mamma-Tumoren erhielten 75 mg/kg/Tag Verbindung Z oral als Futterzusatz; oder 1 $\mu$g/kg/Tag Interleukin-2 i.p. 5 mal wöchentlich; oder beide Testverbindungen. Die Dauer der Behandlung war 10 Wochen. Die Dosierung von IL-2 ist eine ungewöhnlich tiefe Dosierung, die sehr gut vertragen wird. Die Resultate sind in Tabelle 2 und Figur 4 und 5 angegeben:

## Tabelle 2

| Gruppe | Verbindung(en) | Tumor-häufigkeit in der Gruppe [%] | Mittlere Anzahl Tumore/Ratte | Mittlere Tumor-belastung [cm³] | Ueberlebende Ratten [%] | Körpergewicht [g] |
|---|---|---|---|---|---|---|
| **Versuchsbeginn** | | | | | | |
| 1 | Kontrolle | 100 | 3.0 ± 2.0 | 3.6 ± 2.1 | 100 | 287 ± 26 |
| 2 | Z | 100 | 2.7 ± 0.6 | 6.0 ± 5.0 | 100 | 275 ± 4 |
| 3 | IL-2 | 100 | 2.8 ± 0.7 | 3.4 ± 1.2 | 100 | 285 ± 10 |
| 4 | IL-2 + Z | 100 | 2.5 ± 0.6 | 4.0 ± 2.7 | 100 | 293 ± 9 |
| **nach 6 Wochen** | | | | | | |
| 1 | Kontrolle | 100 | 4.0 ± 2.0 | 17.7 ± 11.5 | 100 | 305 ± 30 |
| 2 | Z | 100 | 3.7 ± 1.0 | 14.2 ± 6.5 | 100 | 264 ± 6 |
| 3 | IL-2 | 100 | 3.0 ± 0.5 | 25.4 ± 13.4 | 100 | 296 ± 11 |
| 4 | IL-2 + Z | 83 | 1.5 ± 0.6 | 3.0 ± 2.3 | 100 | 265 ± 13 |
| **nach 10 Wochen** | | | | | | |
| 1 | Kontrolle | 100 | 5.5 ± 3.5 | 18.8 ± 12.4 | 100 | 320 ± 25 |
| 2 | Z | 100 | 3.3 ± 0.5 | 18.2 ± 12.1 | 100 | 261 ± 11 |
| 3 | IL-2 | 100 | 3.5 ± 0.6 | 33.9 ± 17.9 | 100 | 314 ± 13 |
| 4 | IL-2 + Z | 50 | 1.0 ± 0.6 | 2.6 ± 2.4 | 100 | 254 ± 9 |

Figur 4 zeigt, dass die Tumorhäufigkeit bei den mit Z oder IL-2 behandelten Tieren gegenüber der Kontrollgruppe unverändert blieb. Dagegen führte die kombinierte Behandlung mit Z und IL-2 bei drei von sechs Versuchstieren zum völligen Verschwinden der Tumore.

Figur 5 zeigt die mittlere Anzahl der Tumoren unter Behandlung mit Z oder IL-2, unter Behandlung mit Z und IL-2, sowie ohne Behandlung (Kontrollgruppe).

Zu Beginn der Behandlung betrug die mittlere Tumorzahl 3,4 pro Tier. Diese Anzahl verdoppelte sich bei der Kontrollgruppe im Verlauf von 10 Wochen. Die Behandlung mit Z allein führte zu einer Verminderung um 48% in der 5. Woche, danach zu einem leichten Anstieg um etwa 10%. Die Behandlung mit IL-2 allein hatte keinen Einfluss auf die Anzahl der Tumore. Bei der kombinierten Behandlung hatte sich die Anzahl der Tumore nach der 10. Woche um 67,5% vermindert.

C. Behandlung von chemisch induzierten Mamma-Tumoren bei der Ratte mit Doxorubicin und Verbindung Z.

Weibliche Ratten mit wie unter A. beschrieben induzierten Mamma-Tumoren wurden während 12 Wochen mit 75 mg/kg/Tag Verbindung Z als Futterzusatz; oder mit 0,2 mg/kg Doxorubicin i.p. oder mit beiden Testverbindungen behandelt. Die Behandlung erfolgte 5 mal wöchentlich. Die Dosierung von Doxorubicin ist eine ungewöhnliche tiefe Dosis mit sehr guter Verträglichkeit. Die Resultate sind in Tabelle 3 und Figur 6-8 aufgeführt.

Tabelle 3

| Gruppe | Verbindung(en) | Tumor-häufigkeit in der Gruppe [%] | Mittlere Anzahl Tumore/Ratte | Mittlere Tumorbelastung [cm³] | Anzahl der überlebenden Ratten | Körpergewicht [g] |
|---|---|---|---|---|---|---|
| **Versuchsbeginn** | | | | | | |
| 1 | Kontrolle | 100 | 5.2 ± 0.8 | 17.7 ± 6.4 | 7 | 290 ± 10 |
| 2 | Z | 100 | 5.7 ± 0.8 | 17.9 ± 6.4 | 7 | 306 ± 9 |
| 3 | Dox | 100 | 3.8 ± 0.7 | 11.8 ± 7.0 | 7 | 301 ± 16 |
| 4 | Dox + Z | 100 | 4.4 ± 0.6 | 15.0 ± 6.0 | 7 | 292 ± 15 |
| **nach 6 Wochen** | | | | | | |
| 1 | Kontrolle | 100 | 6.5 ± 1.1 | 96.2 ± 31.4 | 7 | 299 ± 14 |
| 2 | Z | 100 | 4.1 ± 0.9 | 14.1 ± 5.4 | 7 | 273 ± 6 |
| 3 | Dox | 100 | 4.2 ± 0.9 | 91.1 ± 54.4 | 7 | 301 ± 9 |
| 4 | Dox + Z | 71 | 2.5 ± 1.0 | 6.6 ± 5.7 | 7 | 254 ± 9 |
| **nach 12 Wochen** | | | | | | |
| 1 | Kontrolle | 100 | 6.4 ± 0.8 | 233.4 ± 105.3 | 5 | 318 ± 12 |
| 2 | Z | 100 | 4.4 ± 0.9 | 36.5 ± 22.2 | 7 | 275 ± 9 |
| 3 | Dox | 100 | 4.5 ± 0.7 | 197.8 ± 132.1 | 6 | 338 ± 28 |
| 4 | Dox + Z | 71 | 1.1 ± 0.4 | 1.2 ± 1.1 | 7 | 251 ± 9 |

Figur 6 zeigt die Tumorhäufigkeit ausgedrückt als Prozentsatz von Tumor-tragenden Tieren in jeder Gruppe. Die Einzelbehandlung mit Z oder Doxorubicin zeigte hier keine Aenderung gegenüber der unbehandelten Kontrollgruppe.

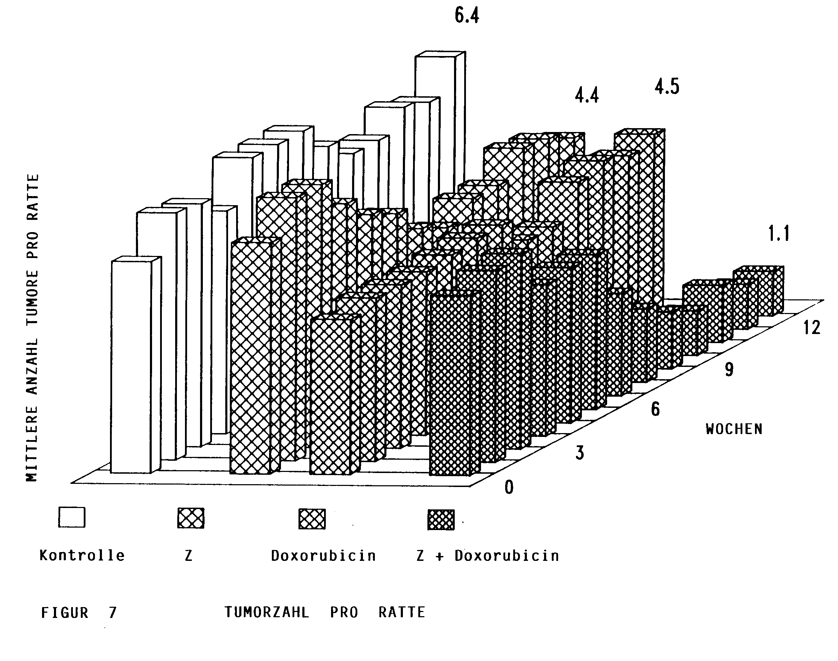

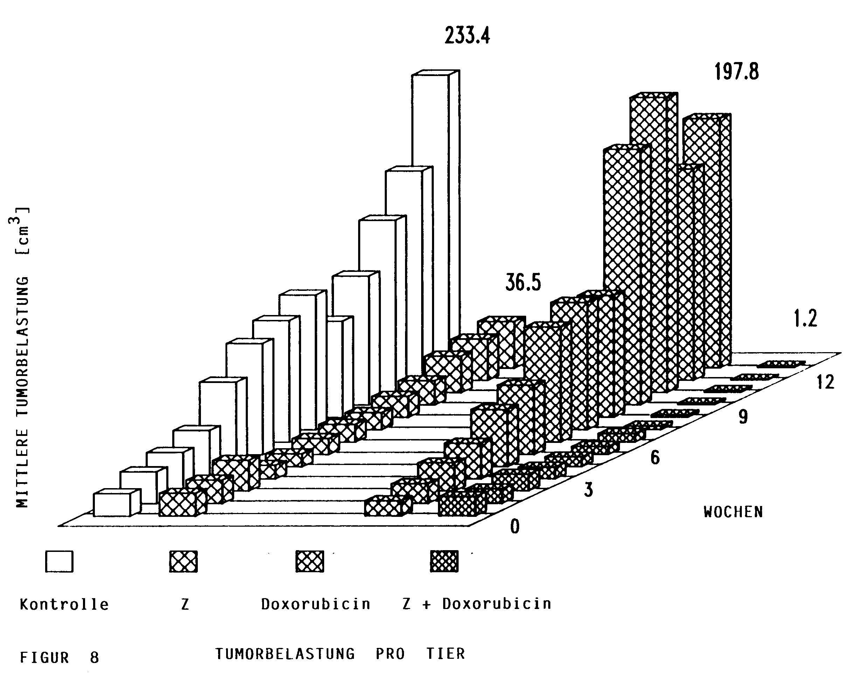

Figur 7 zeigt, dass sich die durchschnittliche Anzahl der Tumore unter Behandlung mit den Einzelpräparaten nur wenig verringerte, die kombinierte Verabreichung zeigte einen synergistischen Effekt. Ein synergistischer Effekt wurde ebenfalls bei dem Parameter "mittlere Tumorbelastung" beobachtet, (Figur 8) der das Produkt von mittlerer Anzahl der Tumore und mittlerem Tumorvolumen darstellt.

D. Antiproliferative Wirkung der Verbindung Z in Kombination mit Interferon auf Human-Tumorzellen in vitro

Für den Test wurden die Human-Mamma-Zell Linien BT-20 (Oestrogenrezeptor-negativ) und ZR 75-1 (Oestrogenrezeptor-positiv) verwendet. Die Zellen wurden in RPMI 1640, das 10% FCS enthielt, kultiviert und auf Gewebskulturplatten ausgesät. Einen Tag nach der Aussaat wurden die Kulturen 14 Tage lang mit der Verbindung Z und Interferon, einzeln und in Kombination, behandelt. Kulturmedium und Testverbindungen wurden alle 2-3 Tage erneuert. Zellzählungen wurden bei Versuchsbeginn, bei jedem Wechsel des Mediums und am Ende des Versuchs vorgenommen. Als Interferone wurden das Humanhybrid rIFN-$\alpha$AD, Humaninterferon rIFN$\alpha_2$ (Wirkstoff von 'Roferon') und rIFN$\gamma$ eingesetzt.

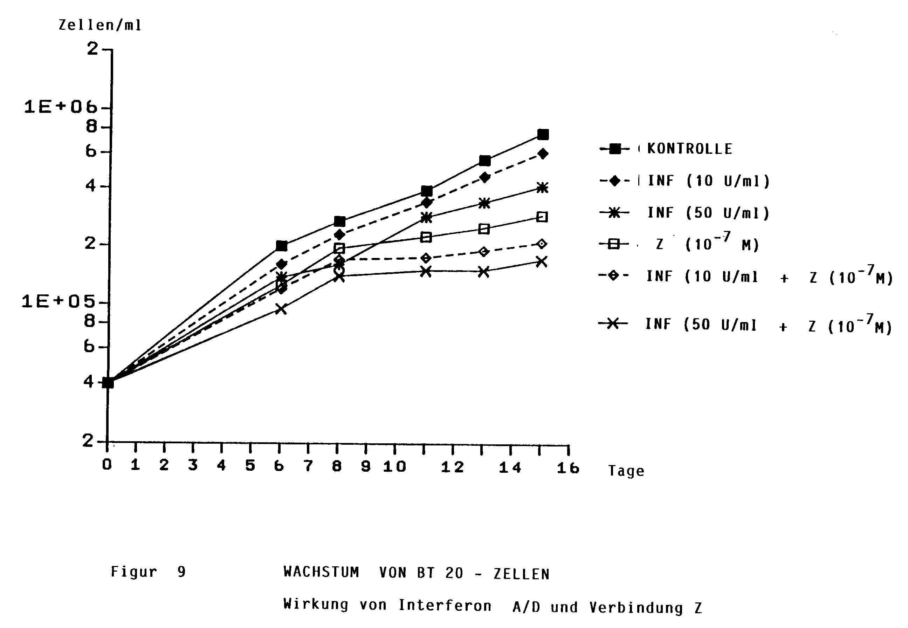

Die Resultate sind in den Figuren 9-12 dargestellt. Die mit IFN$\alpha$A/D und IFN$\alpha_2$ erhaltenen Resultate waren gleich, daher wird für Interferon alpha nur eine Kurve angeführt.

Figur 9 zeigt, dass die bei alleiniger Verabreichung praktisch unwirksame Konzentration von 10 U/ml Interferon die Wirkung der Verbindung Z deutlich verstärkt.

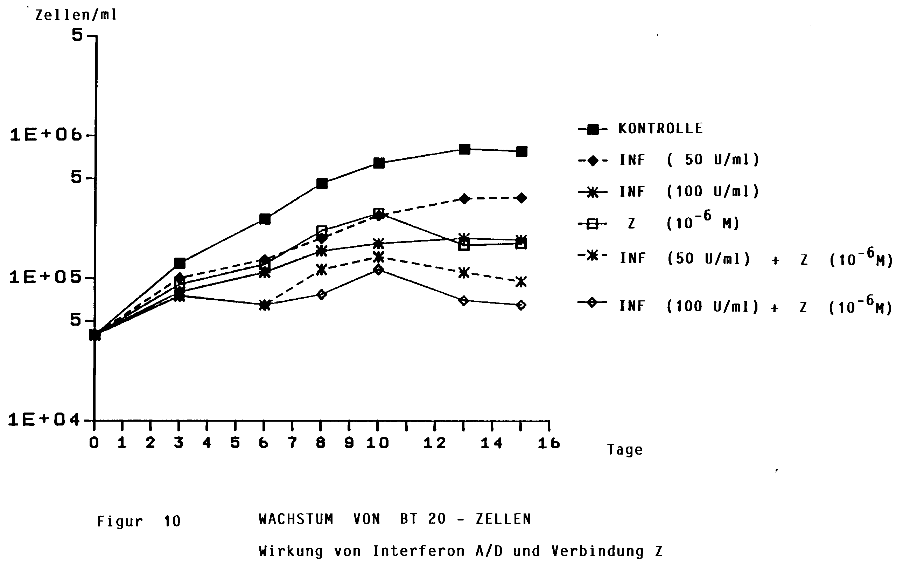

Figur 10 zeigt, dass mit höheren, aber noch untoxischen Dosen der Kombination eine fast vollständige Hemmung der Zellproliferation erreicht wurde.

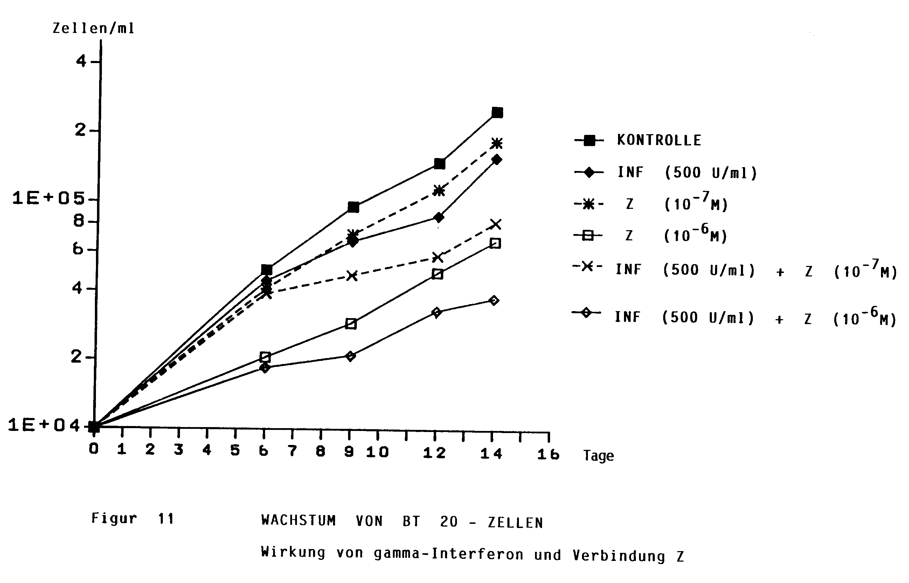

Figur 11 zeigt, dass mit IFN$\gamma$ ähnliche Resultate wie mit IFN$\alpha$A/D erhalten wurden.

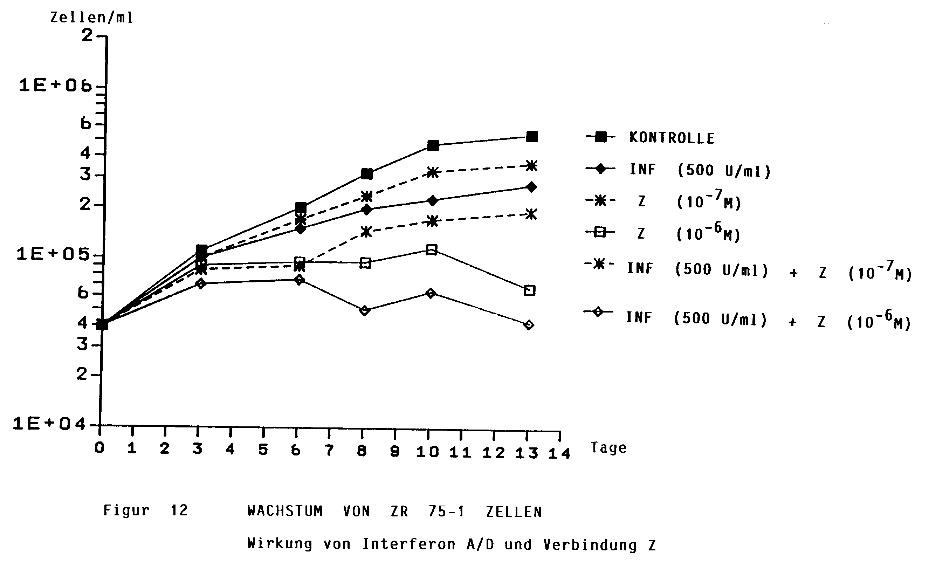

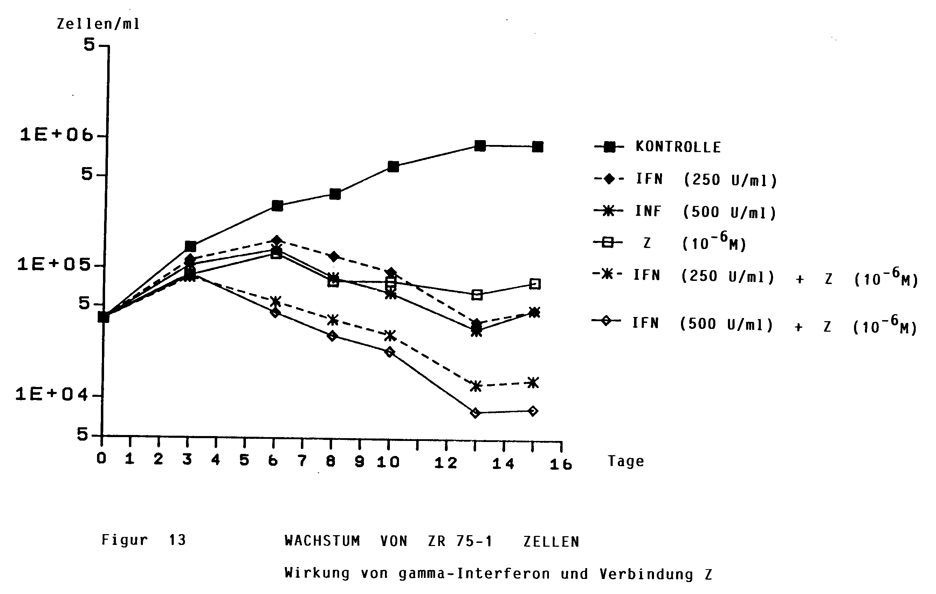

Auch bei der Zell-Linie ZR 75-1 führte die Behandlung mit der Kombination von Verbindung Z und Interferonen zu einer stärkeren Hemmung der Zellproliferation als bei Behandlung mit den Einzelkomponenten (Fig. 12 und 13).

F. Behandlung von humanem bronchialen Plattenepithelkarzinomen (Riedacher LXFE) bei der athymischen Maus

Für diesen Versuch wurde etwa 1 mm$^3$ Tumorgewebe subcutan in athymische Mäuse transplantiert. Die Behandlung (50 mg/kg/Tag Arotinoid p.o.; 50'000 oder 100'000 U/ml Interferon $\alpha$A/D i.p., 5 mal wöchentlich während 4 Wochen) wurde begonnen, sobald die Transplantate auf etwa 0,5 cm Durchmesser gewachsen waren. Das Tumorwachstum wurde wöchentlich durch Ausmessen der grossen und kleinen Durchmesser der Tumore bestimmt. Als positiv-Kontrolle wurde Adriamycin (1 mg/kg/Tag i.p.) eingesetzt.

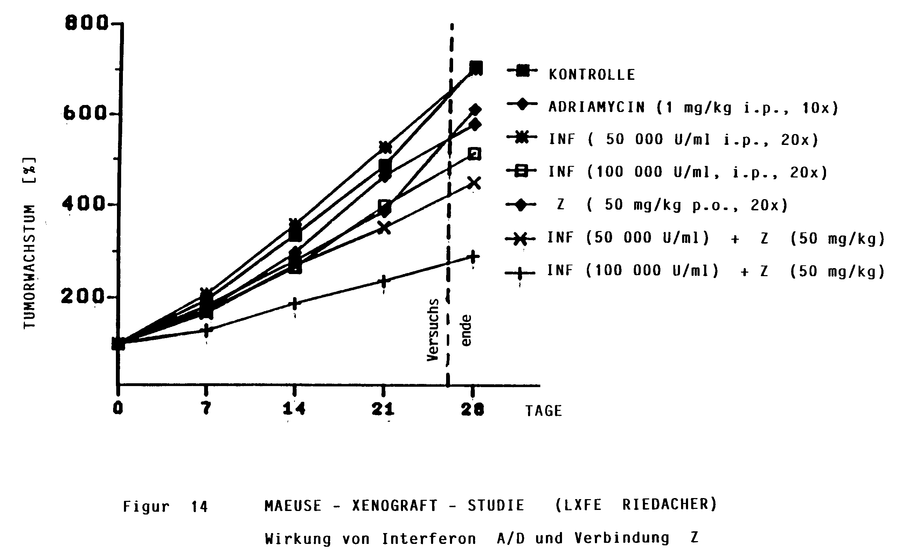

Die Resultate sind in Figur 14 dargestellt. Daraus wird ersichtlich, dass die niedrigere Interferon-Dosis, die für sich allein keinen Einfluss auf das Tumorwachstum hatte, in Kombination mit der Verbindung Z das Tumorwachstum deutlich verminderte.

Die Verbindung Z kann bei der erfindungsgemässen Verwendung in den aus der europäischen Patentpublikation A2-0 331 983 bekannten Dosierungen und Formulierungen eingesetzt werden, wobei die Dosierung im Einzelfall den Bedürfnissen des Patienten und dem parallel eingesetzten weiteren Cytostatikum bzw. BRM angepasst werden muss, was im Rahmen der fachärztlichen Kenntnisse liegt.

**Patentansprüche**

1. Verwendung des 4-[2-[p-[(E)-2-(5,6,7,8-Tetrahydro-5,5,8,8-tetramethyl -2-naphthyl)propenyl]phenoxy]-äthyl]morpholins als Wirkstoff bei der Herstellung von pharmazeutischen Präparaten zur Unterstützung der Therapie mit einem Cytostatikum.

2. Verwendung nach Anspruch 1, wobei das Cytostatikum eine Verbindung vom Typ der alkylierenden Substanzen, der Antimetabolite, der Anthracycline, der Vinca-Alkaloide oder der Biological Response Modifiers ist.

3. Verwendung nach Anspruch 1 oder 2, wobei das Cytostatikum Cyclophosphamid, Doxorubicin, 5-Fluoruracil, Interleukin-2, Interferon alpha, Interferon beta oder Interferon gamma ist.

4. Erzeugnisse, enthaltend 4-[2-[p-[(E)-2-(5,6,7,8-Tetrahydro-5,5,8,8-tetramethyl -2-naphthyl)propenyl]-phenoxy]äthyl]morpholin und ein Cytostatikum als Kombinationspräparat zur gleichzeitigen, getrennten oder zeitlich abgestuften Anwendung in der Krebsprophylaxe oder -Therapie.

5. Handelspackung, enthaltend als pharmazeutischen Wirkstoff 4-[2-[p-[(E)-2-(5,6,7,8-Tetrahydro-5,5,8,8-tetramethyl -2-naphthyl)propenyl]phenoxy]äthyl]morpholin zusammen mit Instruktionen für dessen Verwendung in Kombination mit einem Cytostatikum zur gleichzeitigen, getrennten oder zeitlich abgestuften Anwendung in der Krebsprophylaxe oder -Therapie.

FIGUR  1        PROZENTSATZ  TUMORTRAGENDER  RATTEN  PRO  GRUPPE

EP 0 480 315 A2

FIGUR 2   TUMORBELASTUNG PRO TIER

FIGUR 3          UEBERLEBENSRATE  PRO  GRUPPE

FIGUR 4

FIGUR 5

FIGUR  6                    PROZENTSATZ  TUMORTRAGENDER  RATTEN  PRO  GRUPPE

FIGUR 7          TUMORZAHL  PRO  RATTE

FIGUR 8

TUMORBELASTUNG PRO TIER

MITTLERE TUMORBELASTUNG [cm³]

WOCHEN

233.4

197.8

36.5

1.2

12

9

6

3

0

Kontrolle

Z

Doxorubicin

Z + Doxorubicin

Figur 9        WACHSTUM VON BT 20 - ZELLEN

Wirkung von Interferon A/D und Verbindung Z

EP 0 480 315 A2

Figur 10    WACHSTUM VON BT 20 - ZELLEN

Wirkung von Interferon A/D und Verbindung Z

EP 0 480 315 A2

Figur 11  WACHSTUM VON BT 20 - ZELLEN

Wirkung von gamma-Interferon und Verbindung Z

Figur  12          WACHSTUM  VON  ZR  75-1  ZELLEN

Wirkung von Interferon A/D und Verbindung Z

EP 0 480 315 A2

Figur 13    WACHSTUM VON ZR 75-1 ZELLEN

Wirkung von gamma-Interferon und Verbindung Z

EP 0 480 315 A2

Figur 14    MAEUSE - XENOGRAFT - STUDIE   (LXFE  RIEDACHER)

Wirkung von Interferon  A/D und Verbindung  Z